# EUROPEAN PATENT SPECIFICATION

(11) **EP 0 726 948 B1**
(45) Date of publication and mention of the grant of the patent: **28.02.2007**
(21) Application number: 94930791.2
(22) Date of filing: 14.10.1994
(51) Int. Cl.: C12N 15/12, A61K 48/00, A61K 38/18, C12N 5/06, G01N 33/50

(54) **METHOD OF INDUCING AND MAINTAINING NEURONAL CELLS**
VERFAHREN ZUR INDUZIERUNG UND ZUR ERHALTUNG NEURONALEN ZELLEN
PROCEDE D'INDUCTION ET DE MAINTIEN DE CELLULES NEURONALES

(30) Priority: 14.10.1993 US 136748
(43) Date of publication of application: 21.08.1996
(73) Proprietor: PRESIDENT AND FELLOWS OF HARVARD COLLEGE, Cambridge, Massachusetts 02138 (US)
(72) Inventor: MELTON, Douglas, A., Lexington, MA 02173 (US); HEMMATI-BRIVANLOU, Ali, New York, NY 10028 (US)
(74) Representative: Horner, Martin Grenville
(86) International application number: PCT/US1994/011745
(87) International publication number: WO 1995/010611

(56) References cited:
- NATURE, vol.359, 1992 pages 609 - 614 A. HEMMATI-BRIVANLOU AND D.A. MELTON 'A truncated activin receptor inhibits mesoderm induction and formation of axial structures in Xenopus embryos'
- J. BIOL. CHEM., vol.267, 1992 pages 7203 - 7206 M. HASHIMOTO ET AL. 'Follistatin is a developmentally regulated cytokine in neural differentiation'
- BIOCHEM. BIOPHYS. RES. COMMUN., vol.173, 1990 pages 193 - 200 M. HASHIMOTO ET AL. 'Activin/EDF as an inhibitor of neural differentiation'
- CELL, vol.77, 1994 pages 273 - 281 A. HEMMATI-BRIVANLOU AND D.A. MELTON 'Inhibition of activin receptor signaling promotes neuralization in Xenopus'
- CELL, vol.77, 1994 pages 283 - 295 A. HEMMATI-BRIVANLOU ET AL. 'Follistatin, an antagonist of activin, is expressed in the Spemann organizer and displays direct neuralizing activity'

### SEQUENCE LISTING

(1) GENERAL INFORMATION:
   (i) APPLICANT:
      (A) NAME: President and Fellows of Harvard College
      (B) STREET: 124 Mt. Auburn Street
      (C) CITY: Cambridge
      (D) STATE: MA
      (E) COUNTRY: USA
      (F) POSTAL CODE (ZIP): 02138
      (G) TELEPHONE: (617) 227-7400
      (H) TELEFAX: (617) 227-5941
   (ii) TITLE OF INVENTION: Method of Inducing and Maintaining Neuronal Cells
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: ASCII (text)
   (v) CURRENT APPLICATION DATA: APPLICATION NUMBER: US 08/136,748
(2) INFORMATION FOR SEQ ID NO:1:
   (i) SEQUENCE CHARACTERISTICS:
      (A) LENGTH: 1178 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 108..1067
   (xi) SEQUENCE DESCRIPTION: SEQ ID NO:1:
   GCGGCCGCCC CCCCCCCCGT CATTCAATAG AGTCCGGACT TGTGCCTGGT CCATTATCCC        60
   ATCTCTCTCC ACTTGAGACT CTGCTCGTCC CACTCCCAGC ACTGAGG ATG TTA AAT      116
                                                       Met Leu Asn
                                                         1
   GAA AGG ATC CAG CCG GGC ATG ATT TTC CTC CTG ACT GTC TCC CTG TGC      164
   Glu Arg Ile Gln Pro Gly Met Ile Phe Leu Leu Thr Val Ser Leu Cys
         5                  10                  15
   CAT TTC ATG GAA TAT CGC GCA GTC CAA GCT GGG AAT TGC TGG CTG CAG      212
   His Phe Met Glu Tyr Arg Ala Val Gln Ala Gly Asn Cys Trp Leu Gln
    20                 25                  30                  35
   CAG TCG AAG AAT GGC CGA TGT CAG GTT CTG TAC AGG ACA GAA CTG AGC      260
   Gln Ser Lys Asn Gly Arg Cys Gln Val Leu Tyr Arg Thr Glu Leu Ser
                   40                  45                  50
   AAA GAG GAA TGC TGC AAG ACT GGC AGA CTG GGC ACC TCA TGG ACA GAA      308
   Lys Glu Glu Cys Cys Lys Thr Gly Arg Leu Gly Thr Ser Trp Thr Glu
               55                  60                  65
   GAA GAT GTA CCC AAC AGC ACC CTC TTC AAA TGG ATG ATA TTT CAT GGA      356
   Glu Asp Val Pro Asn Ser Thr Leu Phe Lys Trp Met Ile Phe His Gly
           70                  75                  80
   GGG GCC CCA CAT TGC ATC CCC TGC AAA GAA ACA TGT GAG AAC GTA GAC      404
   Gly Ala Pro His Cys Ile Pro Cys Lys Glu Thr Cys Glu Asn Val Asp
       85                  90                  95
   TGT GGC CCT GGG AAG AAA TGT AAA ATG AAC AAG AAG AAC AAG CCG AGG      452
   Cys Gly Pro Gly Lys Lys Cys Lys Met Asn Lys Lys Asn Lys Pro Arg
   100                 105                 110                 115
   TGT GTC TGC GCT CCG GAT TGT TCC AAC ATT ACT TGG AAA GGT TCA GTG      500
   Cys Val Cys Ala Pro Asp Cys Ser Asn Ile Thr Trp Lys Gly Ser Val
                   120                 125                 130
   TGC GGA ATT GAT GGC AAA ACC TAT AAG GAT GAG TGC GCT TTG CTC AAA      548
   Cys Gly Ile Asp Gly Lys Thr Tyr Lys Asp Glu Cys Ala Leu Leu Lys
               135                 140                 145
   GCC AAA TGT AAA GGG GTC CCG GAG CTG GAT GTG CAG TAC CAA GGA AAA      596
   Ala Lys Cys Lys Gly Val Pro Glu Leu Asp Val Gln Tyr Gln Gly Lys
           150                 155                 160
   TGC AAA AAG ACT TGC AGG GAC GTG CTG TGT CCA GGG AGC TCC TCG TGT      644
   Cys Lys Lys Thr Cys Arg Asp Val Leu Cys Pro Gly Ser Ser Ser Cys
       165                 170                 175
   GTG GTG GAT CAG ACC AAT AAC GCC TAC TGT GTG ACA TGT AAT CGG ATT      692
   Val Val Asp Gln Thr Asn Asn Ala Tyr Cys Val Thr Cys Asn Arg Ile
   180                 185                 190                 195
   TGC CCG GAG CCT ACC TCC CCT GAC CAA TAT CTG TGT GGG AAT GAT GGA      740
   Cys Pro Glu Pro Thr Ser Pro Asp Gln Tyr Leu Cys Gly Asn Asp Gly
                   200                 205                 210
   ATA ACC TAT GGA AGT GCG TGC CAC CTG AGG AAG GCT ACC TGC CTG CTG      788
   Ile Thr Tyr Gly Ser Ala Cys His Leu Arg Lys Ala Thr Cys Leu Leu
               215                 220                 225
   GGC AGA TCC ATT GGA TTA GCC TAC GAG GGG AAA TGC ATA AAA GCC AAG      836
   Gly Arg Ser Ile Gly Leu Ala Tyr Glu Gly Lys Cys Ile Lys Ala Lys
           230                 235                 240
   TCT TGT GAA GAT ATT CAG TGC AGC GCT GGA AAG AAA TGC CTG TGG GAC      884
   Ser Cys Glu Asp Ile Gln Cys Ser Ala Gly Lys Lys Cys Leu Trp Asp
       245                 250                 255
   AGT AGA GTG GGT AGA GGT CGC TGT GCG CTG TGC GAT GAT CTG TGC GGA          932
   Ser Arg Val Gly Arg Gly Arg Cys Ala Leu Cys Asp Asp Leu Cys Gly
   260                 265                 270                 275
   GAG AGC AAG TCA GAC GAT ACA GTG TGC GCC AGC GAC AAC ACG ACT TAC          980
   Glu Ser Lys Ser Asp Asp Thr Val Cys Ala Ser Asp Asn Thr Thr Tyr
                   280                 285                 290
   CCG AGC GAG TGC GCC ATG AAA CAG GCA GCC TGC TCC ACG GGG ATT CTT         1028
   Pro Ser Glu Cys Ala Met Lys Gln Ala Ala Cys Ser Thr Gly Ile Leu
               295                 300                 305
   TTG GAA GTG AAA CAC AGT GGA TCT TGC AAC TGT AAG TGAATTACCG              1074
   Leu Glu Val Lys His Ser Gly Ser Cys Asn Cys Lys
           310                 315                 320
   CAACGCAGAG TAAGATTTCT AAAGGCAACC CCTCGGTAAT GAAGACTTTA AAGCAGCAAA       1134
   ATACTTTTTT TTTTTTTTTT TCCTTTTTTT CTAAGGGAAT TCAG                        1178
(2) INFORMATION FOR SEQ ID NO:2:
   (i) SEQUENCE CHARACTERISTICS:
      (A) LENGTH: 319 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Leu Asn Glu Arg Ile Gln Pro Gly Met Ile Phe Leu Leu Thr Val
    1               5                  10                  15
   Ser Leu Cys His Phe Met Glu Tyr Arg Ala Val Gln Ala Gly Asn Cys
               20                  25                  30
   Trp Leu Gln Gln Ser Lys Asn Gly Arg Cys Gln Val Leu Tyr Arg Thr
           35                  40                  45
   Glu Leu Ser Lys Glu Glu Cys Cys Lys Thr Gly Arg Leu Gly Thr Ser
       50                  55                  60
   Trp Thr Glu Glu Asp Val Pro Asn Ser Thr Leu Phe Lys Trp Met Ile
   65                  70                  75                  80
   Phe His Gly Gly Ala Pro His Cys Ile Pro Cys Lys Glu Thr Cys Glu
                   85                  90                  95
   Asn Val Asp Cys Gly Pro Gly Lys Lys Cys Lys Met Asn Lys Lys Asn
               100                 105                 110
   Lys Pro Arg Cys Val Cys Ala Pro Asp Cys Ser Asn Ile Thr Trp Lys
           115                 120                 125
   Gly Ser Val Cys Gly Ile Asp Gly Lys Thr Tyr Lys Asp Glu Cys Ala
       130                 135                 140
   Leu Leu Lys Ala Lys Cys Lys Gly Val Pro Glu Leu Asp Val Gln Tyr
   145                 150                 155                 160
   Gln Gly Lys Cys Lys Lys Thr Cys Arg Asp Val Leu Cys Pro Gly Ser
                   165                 170                 175
   Ser Ser Cys Val Val Asp Gln Thr Asn Asn Ala Tyr Cys Val Thr Cys
               180                 185                 190
   Asn Arg Ile Cys Pro Glu Pro Thr Ser Pro Asp Gln Tyr Leu Cys Gly
           195                 200                 205
   Asn Asp Gly Ile Thr Tyr Gly Ser Ala Cys His Leu Arg Lys Ala Thr
       210                 215                 220
   Cys Leu Leu Gly Arg Ser Ile Gly Leu Ala Tyr Glu Gly Lys Cys Ile
   225                 230                 235                 240
   Lys Ala Lys Ser Cys Glu Asp Ile Gln Cys Ser Ala Gly Lys Lys Cys
                   245                 250                 255
   Leu Trp Asp Ser Arg Val Gly Arg Gly Arg Cys Ala Leu Cys Asp Asp
               260                 265                 270
   Leu Cys Gly Glu Ser Lys Ser Asp Asp Thr Val Cys Ala Ser Asp Asn
           275                 280                 285
   Thr Thr Tyr Pro Ser Glu Cys Ala Met Lys Gln Ala Ala Cys Ser Thr
       290                 295                 300
   Gly Ile Leu Leu Glu Val Lys His Ser Gly Ser Cys Asn Cys Lys
   305                 310                 315

## Description

### Background of the Invention

Understanding the processes that lead from a fertilized egg to the formation of germ layers and subsequently to a body plan is a central goal of embryology. Much of what is known about the development of a vertebrate body plan comes from studies of amphibia where, at the tadpole stage, the main body axis consists of the dorsal structures notochord, spinal cord and somites organized anterior to posterior as head, trunk and tail. All animal tissues derive from the three germ layers and the mesoderm plays a pivotal role in organizing the body axis (Keller, R. in *Methods in Cell Biology,* eds Kay and Peng, Academic Press: San Diego, 1991). Mesodermal cells lead the movements of gastrulation (Keller et al. (1988) *Development* 103:193-210; and Wilson et al. (1989) *Development* 105:155-166), are required for the patterning of the nervous system (Mangold et al. (1933) *Natyrwissenschaften* 21*:*761-766; and Hemmati-Brivanlou et al. (1990) *Science* 250:800-802), and themselves give rise to the muscular, skeletal, circulatory and excretory systems. Moreover, a portion of the dorsal mesoderm from early gastrula, the Spemann organizer, can induce and organize a second body axis following transplantation to another site (Spemann et al. (1924) *Arch mikr Anat EntwMech* 100:599-638).

The origin of the nervous system in all vertebrates can be traced to the end of gastrulation. At this time, the ectoderm in the dorsal side of the embryo changes its fate from epidermal to neural. The newly formed neuroectoderm thickens to form a flattened structure called the neural plate which is characterized, in some vertebrates, by a central groove (neural groove) and thickened lateral edges (neural folds). At its early stages of differentiation, the neural plate already exhibits signs of regional differentiation along its anterior posterior (A-P) and mediolateral axis (M-L). The neural folds eventually fuse at the dorsal midline to form the neural tube which will differentiate into brain at its anterior end and spinal cord at its posterior end. Closure of the neural tube creates dorsal/ventral differences by virtue of previous mediolateral differentiation. Thus, at the end of neurulation, the neural tube has a clear anterior-posterior (A-P), dorsal ventral (D-V) and mediolateral (M-L) polarities (see, for example, *Principles in Neural Science (3rd),* eds. Kandel, Schwartz and Jessell, Elsevier Science Publishing Company: NY, 1991; and *Developmental Biology (3rd),* ed. S.F. Gilbert, Sinauer Associates: Sunderland MA, 1991).

Before gastrulation the three germ layers are simply arranged, top to bottom, in a frog blastula. Ectoderm arises from the top, or animal pole; mesoderm from the middle, or marginal zone, and endoderm from the bottom or vegetal pole. Mesoderm can be induced in animal pole cells (animal caps) by signals emanating from the vegetal pole. Several peptide growth factors have been identified that can induce mesoderm in animal caps *in vitro.* When animal cap tissue is explanted from a blastula embryo and cultured in isolation it develops into a ball of epidermis. But in the presence of a mesoderm inducing factor, the animal cap will differentiate into mesodermal derivatives, including notochord, muscle and blood. Members of the fibroblast growth factor family, in particular basic fibroblast growth factor (bFGF), and the transforming growth factor-β (TGF-β) family, notably activins and Vg-1, are potent inducers in this assay. Xenopus homologues of the Wnt gene family may also have a role in mesoderm induction. Both Xwntl (McMahon et al. (1989) *Cell* 58, 1075-1084) and Xwnt8 messenger RNAs elicit dorsal mesoderm formation when injected into the ventral side of an early embryo, an activity shared by Vg-1, and to a lesser extent by activin RNA. bFGF and activin protein' can be detected in the early embryo and although there are no data on the localization of activin, there is evidence that bFGF is present in the marginal zone and vegetal pole of early blastula. Vg-1 is present at the appropriate time and in the right region known to be responsible for mesoderm induction *in vivo.* Although Xwntl and Xwnt8 are not present at the proper time or place to effect dorsal mesoderm induction, there may be other Xwnts that fulfill this role.

Many types of communication take place among animal cells. These vary from long-range effects, such as those of rather stable hormones circulating in the blood and acting on any cells in the body that possess the appropriate receptors, however distant they are, to the fleeting effects of very unstable neurotransmitters operating over distances of only a few microns. Of particular importance in development is the class of cell interactions called embryonic induction; this includes influences operating between adjacent cells or in some cases over greater than 10 cell diameters (Saxen et al. (1989) *Int J Dev Biol* 33:21-48; and Gurdon et al. (1987) *Development* 99:285-306). Embryonic induction is defined as in interaction between one (inducing) and another (responding) tissue or cell, as a result of which the responding cells undergo a change in the direction of differentiation. This interaction is often considered one of the most important mechanism in vertebrate development leading to differences between cells and to the organization of cells into tissues and organs. Adult organs in vertebrates, and probably in invertebrates, are formed through an interaction between epithelial and mesenchymal cells, that is, between ectoderm/endoderm and mesoderm, respectively.

The effects of developmental cell interactions are varied. Typically, responding cells are diverted from one route of cell differentiation to another, by inducing cells that differ from both the uninduced and induced states of the responding cells (inductions). Sometimes cells induce their neighbors to differentiate like themselves (homoiogenetic induction); in other cases a cell inhibits its neighbors from differentiating like itself. Cell interactions in early development may be sequential, such that an initial induction between two cell types leads to a progressive amplification of diversity. Moreover, inductive interactions occur not only in embryos, but in adult cells as well, and can act to establish and maintain morphogenetic patterns as well as induce differentiation (J.B. Gurdon (1992) *Cell* 68:185-199).

While there has been considerable progress in identifying molecules responsible for mesoderm induction, practically nothing is known about the molecular nature of neural induction. Candidate neural patterners are growth factors that are involved in mesoderm patterning in earlier stages and become localized later in a subset of cells in the nervous system. These molecules include different members of the Wnt, TGF-β and FGF families. Three members of the Wnt family Wnt-1, Wnt-3 and Wnt-3A, are localized in the roof plate (dorsal spinal cord) and a subset of brain cells. Good evidence that Wnt products pattern the neural tube comes from homozygote mice lacking the Wnt-1 gene product; these mutant mice display a strong abnormality in the anterior hindbrain and posterior midbrain (a region that coincides with engrailed-2 expressing cells)(McMahon et al. (1992) Cell. 69:581-595). Vg-1, BMP-4 (Jones et al. (1991) *Development.* 111:532-542) and dorsalin-1 (Blumberg et al. (1991) *Science* 253:194-196) are examples or TGF-β family members that display restricted expression in the embryonic nervous system (see also, Lyons et al. (1991) *Trends Genet* 7:408-412; and Massague et al. (1990) *J Biol Chem* 265:21393-21396). Dorsalin-1 inhibits the differentiation of motor neurons and induces migration of neural crest cells and thus may be involved in dorsal ventral patterning of the neural tube (Blumberg et al. (1991) *Science* 253:194-196). Finally acidic FGF (aFGF), basic FGF (bFGF) as well as the newly characterized FGF from Xenopus embryos, XeFGF, (Isaacs et al. (1992) Development. 114:711-20) are all expressed in some cells of the developing neural tube (Weise et al. (1992) *Cell & Tissue Research.* 276:125-130; and Tannahill et al. (1992) *Development.* 115:695-702). In addition, Hemmati-Brivanlou & Melton (1992) *Nature.* 359:609-614, describe the effect of a truncated activin receptor in a *Xenopus* system, Hashimoto *et al.,* (1992) *J Biol Chem* 267:7203-7206, details the effects of follistatin on transformed cell lines and Van den Eijndenvan Raaij *et al.,* (1992) *Dev Biol* 154(2):356-365, investigate the expression profiles of follistatin in proliferating mouse embryonic stem cells and cells derived from differentiated mouse P19 embryonal carcinoma (EC) cells.

Since the natural embryonic neural inducer or patterner has yet to be characterized, the analysis of the mechanisms of induction and patterning is difficult. However, studies have demonstrated that notochord can induce and pattern neural structures (Jones et al. (1989) *Development.* 107:785-791; and Sharpe et al. (1987) *Cell.* 50:749-758) which implies that the signals can travel vertically from the axial mesoderm to the overlying ectoderm. The finding that neuralization can be induced by mesoderm suggests that neural induction involves a signal acting in a paracrine fashion, the transduction of which appears to involve protein kinase C (Otte et al. (1991) *Science.* 251:570-573). A recent series of experiments, exploring one of Spemann's original ideas, have demonstrated that signals involved in both induction and patterning of the nervous system can also travel through the plane of the ectoderm (Dixon et al. (1989) *Development* 106:749-757: Doniach et al. (1992) *Science* 257:542-545; and Ruiz i Altaba, A. (1992) *Development.* 115:67-80). It is now accepted that both types of mechanisms coexist in the embryo and play a role in neurogenesis.

### Summary of the Invention

In a first aspect, the present invention provides a method for inducing a stem cell to differentiate to a neuronal cell phenotype *in vitro,* comprising contacting said cell with an agent that antagonizes the biological action of activin, said activin normally inducing said cell to differentiate to a non-neuronal phenotype, wherein said agent is a follistatin protein and wherein said stem cell is other than a human embryonic cell. The present invention makes available a method for inducing neuronal differentiation and preventing the death and/or degeneration of neuronal cells both in vitro. The subject method stems from the unexpected finding that, contrary to traditional understanding of neural induction, the default fate of ectodermal tissue is neuronal rather than mesodermal and/or epidermal. In particular, it has been discovered that preventing or antagonizing a signaling pathway in a cell for a growth factor of the TGF-β family (hereinafter "TGF-β-type growth factor"), can result in neuronal differentiation of that cell. In the subject method, signaling by the TGF-β-type growth factor is disrupted by antagonizing the inhibitory activity of the TGF-β-type growth factor. For instance, this can be accomplished by sequestering the growth factor with a growth factor binding protein (such as an activin binding protein where the neural-inhibitory growth factor is activin) or by treating with an antagonist which competes with the growth factor for binding to a growth factor receptor on the surface of the cell of interest.

In one embodiment of the subject method, inducing cells to differentiate to a neuronal cell phenotype comprises contacting the cells in vitro with an agent which antagonizes the biological action of at least one polypeptide growth factor of the TGF-β family which normally induces the cells to differentiate to a non-neuronal phenotype. The antagonizing agent can inhibit the biological activity of the TGF-β-type growth factor, for example, by preventing the growth factor from binding its receptors on the surface of the treated cells. In another embodiment, the antagonizing agent binds to the growth factor and sequesters the growth factor such that it cannot bind its receptors.

To further illustrate the invention, the antagonizing agent is follistatin,

In such instances, the method comprises contacting the cells in vitro with an agent which disrupts the activin signaling pathway in the cells, causing the cells to default to neuronal differentiation, rather than, for instance, mesodermal and/or epidermal fates.

The present method can be used *in vitro,* for example, to induce cells in culture to differentiate to a neuronal phenotype. Moreover, follistatin is amenable to therapeutic application, and as described below, can be used in the preparation of a medicament to treat neurodegenerative disorders associated with, for example, the progressive and persistent loss of neuronal cells, such as which occurs with Alzheimer's disease, Parkinson's disease, amyotrophic lateral sclerosis, Pick's disease, Huntington's disease, multiple sclerosis, neuronal damage resulting from anoxia-ischemia, neuronal damage resulting from trauma, and neuronal degeneration associated with a natural aging process.

### Detailed Description Of The Invention

As described herein, a collective group of experiments performed with recombinant follistatin establish that a signaling pathway of a growth factor of the TGF-β family is involved in inhibiting neural induction *in vivo.* The present findings indicate, for the first time in vertebrates, that neuralization is a default state. As described in the Examples below, our results indicate that activin, or any other member of the TGF-β family that interacts with the truncated activin receptor, can inhibit neural induction, as these TGF-β signals instruct cells towards non-neuronal facts such as epidermal, mesodermal or endodermal fate. Inhibition of signal transduction by a TGF-β-type growth factor, by follistatin, induced cells of the intact animal cap to switch to a neuronal fate in the absence of any detectable mesoderm. This data indicates that presumptive neural tissue in animal caps can respond to TGF-β-type growth factor and form mesodermal and/or epidermal tissues, but if this tissue specification by the factor is blocked, the cells become neural. As described below, activin is strongly implicated as the TGF-β-type growth factor that inhibits neuronal differentiation. Both activin and its receptor are present naturally in the animal cap, indicating that an endogenous activity that blocks activin signaling switches the tissue from an ectodermal to a neural fate. Thus, endogenous activin may act as a neural inhibitor (acting to induce epidermal development and/or mesoderm), and neuralization requires the inhibition of activin activity.

The present invention makes available a method for inducing neuronal differentiation and/or preventing the death or degeneration of neuronal cells. In general, the method comprises contacting a cell, *in vitro,* with an agent capable of antagonizing the bioligical action of a protein from the family of transforming growth factor-βs. The mechanism of action of the antagonist can, for example, comprise: competitive or non-competitive binding to a cell-surface receptor for the growth factor; sequestration of the growth factor; or inhibition of signal transduction events mediated by the growth factor receptor. Representative embodiments are described in more detail below.

The subject method stems from the unexpected finding that, contrary to traditional understanding of neural induction, the default fate of ectodermal tissue is neuronal rather than epidermal. In particular, it has been discovered that preventing or antagonizing a TGF-β-type growth factor signaling pathway for a cell can result in neuronal differentiation of that cell. In the subject method, signaling by the TGF-β-type growth factor is disrupted by antagonizing the inhibitory activity of the TGF-β-type growth factor. For instance, this can be accomplished by sequestering the growth factor with a growth factor binding protein (such as an activin-binding protein) or by treating with an antagonist which competes with the growth factor for binding to a growth factor receptor on the surface of the cell of interest.

As described herein, the present method can be used *in vitro,* for example, to induce cells in culture to differentiate to a neuronal phenotype. In one embodiment, the differentiated cells are subsequently continued in culture, and can be used to provide useful *in vitro* assay systems as well as valuable research tools for further understanding neural development. The differentiated cells may be used *in vivo* for transplantation. Moreover, follistatin is amenable to therapeutic application, and as described below, can be used in the preparation of a medicament to treat neurodegenerative disorders associated with, for example, the progressive and persistent loss of neuronal cells, such as which occurs with Parkinson's disease, Alzheimer's disease, amyotrophic lateral sclerosis, and Huntington's disease.

While the following description, for clarity, describes the use of agents which antagonize activin signaling, it is understood that many such agents can also bind or otherwise antagonize other TGF-β-type growth factors and thereby disrupt their inhibition, if any, of neuralization. As used herein, the terms "transforming growth factor-beta" and "TGF-β" denote a family of structurally related paracrine polypeptides found ubiquitously in vertebrates, and prototypic of a large family of metazoan growth, differentiation, and morphogenesis factors (see, for review, Massaque et al. (1990) Ann *Rev Cell Biol* 6:597-64I; and Sporn et al. (1992) *J Cell Biol* 119:1017-1021). Moreover, the present invention, namely the discovery that neuralization is a default state, will readily allow identification of other factors, including other TGF-β-like growth factors, which inhibit a cell from reaching this default (e.g. actively induce non-neuronal differentiation). In light of this understanding, agents which disrupt these factors are specifically contemplated by the present invention.

An agent capable of antagonizing the signaling pathway of a TGF-β factor involved in preventing neuronal differentiation, so as to cause a cell to default to neuronal differentiation, is herein referred to as a neuralizing agent, or "NA".

In one embodiment, the NA is an activin-binding protein which can reduce the bioavailability of activin, e.g. by sequestering activin in the extracellular milleu, with exemplary activin-binding agents being follistatins. Other activin-binding proteins can include agrin, agrin-related proteins, and other proteins containing follistatin modules.

In an illustrative embodiment of the present method, the NA is a follistatin able to bind and sequester activin. Follistatins are single chain, glycosylated polypeptides that were first isolated based on their ability to inhibit follicle-stimulating hormone release. Follistatins from several species, including human, have been structurally characterized and cloned (See, for example, Esch et al. (1987) *Mol. Endocrinology* 11:849; Ling et al. International Publication No. WO 89/01945; Ling et al. U.S. Patent Serial No. 5,182,375; Ling et al. U.S. Patent Serial No. 5,041,538; and Inouye et al. (1991) *Endocrinology* 129:815-822). For instance, two forms of human follistatin have been cloned and expressed, one having 315 amino acid residues, and one having 288 amino acid residues. (Inouye et al., supra). Human follistatin is available through the National Hormone and Pituitary Program of the NIH. In a one embodiment, the follistatin of the present method is of the class of shorter follistatins (*e.g*. the 288 a.a. human homolog), since, as described below, these forms appear to have a greater binding affinity for activin, relative to the larger forms of follistatin.

Another aspect of the present invention relates to an in vitro method of inducing and/or maintaining a differentiated state, and/or enhancing survival of a neural cell responsive to activin induction, by contacting the cell with a neuralizing agent (e.g. an activin antagonist). For instance, it is contemplated by the invention that, in light of the present finding of an apparently broad involvement of activin in the formation of ordered spatial arrangements of differentiated neural tissues in vertebrates, the subject method could be used to generate and/or maintain an array of different neural tissue *in vitro*.

For example, the present method is applicable to cell culture technique. *In vitro* neuronal culture systems have proved to be fundamental and indispensable tools for the study of neural development as well as the identification of neurotrophic factors such as nerve growth factor (NGF), ciliary trophic factors (CNTF), and brain derived neurotrophic factor (BDNF). Once a neuronal cell has become terminally-differentiated it typically will not change to another terminally differentiated cell-type. However, neuronal cells can nevertheless readily lose their differentiated state. This is commonly observed when they are grown in culture from adult tissue, and when they form a blastema during regeneration. The present method provides a means for ensuring an adequately restrictive environment in order to maintain neuronal cells at various stages of differentiation, and can be employed, for instance, in cell cultures designed to test the specific activities of other trophic factors. In such embodiments of the subject method, the non-human embryonic cultured cells can be contacted with a neuralizing agent of the present invention in order to induce neuronal differentiation (e.g. of a stem cell), or to maintain the integrity of a culture of terminally-differentiated neuronal cells by preventing loss of differentiation. The source of the neuralizing agent in the culture can be derived from, for example, a purified or semi-purified protein composition added directly to the cell culture media, or alternatively, released from a polymeric device which supports the growth of various neuronal cells and which has been doped with the neuralizing agent. If appropriate, the source of the neuralizing agent can also be a cell that is co-cultured with the intended neuronal cell and which produces a recombinant neuralizing agent. Alternatively, the source can be the neuronal cell itself which as been engineered to produce a recombinant neuralizing agent. In an exemplary embodiment, a non-human embryonic naive neuronal cell (e.g. a stem cell) is treated with an activin antagonist in order to induce differentiation of the cells into, for example, sensory neurons or, alternatively, motorneurons, Such neuronal cultures can, be used as convenient assay systems as well as sources of implantable cells for the preparation of medicaments

To further illustrate potential uses, it is noted that intracerebral grafting has emerged as an additional approach to central nervous system therapies. For example, one approach to repairing damaged brain tissues involves the transplantation of cells from fetal or neonatal animals into the adult brain (Dunnett et al. (1987) *J Exp Biol* 123:265-289; and Freund et al. *(1985) J Neurosci* 5:603-616). Fetal neurons from a variety of brain regions can be successfully incorporated into the adult brain, and such grafts can alleviate behavioral defects. For example, movement disorder induced by lesions of dopaminergic projections to the basal ganglia can be prevented by grafts of non-human embryonic dopaminergic neurons. Complex cognitive functions that are impaired after lesions of the neocortex can also be partially restored by grafts of non-human embryonic cortical cells. Thus, use of activin antagonist for maintenance of neuronal cell cultures can help to provide a source of implantable neuronal tissue. The use of a neuralizing agent in the culture can be to prevent loss of differentiation, or where fetal tissue is used, especially neuronal stem cells, a neuralizing agent of the present invention can be used to induce differentiation.

Non-human embryonic stem cells useful in the present invention are generally known. For example, several neural crest cells have been identified, some of which are multipotent and likely represent uncommitted neural crest cells, and others of which can generate only one type of cell, such as sensory neurons, and likely represent committed progenitor cells. The role of an activin-disrupting agent employed in the present method to culture such stem cells can be to induce differentiation of the uncommitted progenitor and thereby give rise to a committed progenitor cell, or to cause further restriction of the developmental fate of a committed progenitor cell towards becoming a terminally-differentiated neuronal cell. For example, the present method can be used *in vitro* to induce and/or maintain the differentiation of neural crest cells into glial cells, schwann cells, chromaffin cells, cholinergic sympathetic or parasympathetic neurons, as well as peptidergic and serotonergic neurons. The neuralizing agent can be used alone, or can be used in combination with other neurotrophic factors which act to more particularly enhance a particular differentiation fate of the neuronal progenitor cell. In the later instance, the neuralizing agent might be viewed as ensuring that the treated cell has achieved a particular phenotypic state such that the cell is poised along a certain developmental pathway so as to be properly induced upon contact with a secondary neurotrophic factor. In similar fashion, even relatively undifferentiated non-human embryonic stem cells or primative neuroblasts can be maintained in culture and caused to differentiate by treatment with the subject neuralizing agents. Exemplary primative cell cultures comprise cells harvested from the nueral plate or neural tube of a non-human embryo even before much overt differentiation has occurred.

The method of the present invention will also facilitate further determination of a potential role of follistatin as a "morphogen", that is, a molecule whose tight threshold of concentration determines specific cell fate during development (Wolpert, L. (1969) *J*. *Theor Biol.* 25:1-47). One of the first molecules to qualify as a morphogen was bicoid, a DNA binding protein whose graded distribution in the syncytium of the Drosophila embryo leads to the generation of specific cell fates (Driever et al. (1988) *Cell* 54:95-104). More recently two factors, activin in Xenopus embryos (Green et al. (1992) *Cell* 71:731-739) and decapentaplegic (dpl), (Ferguson et al. (1992) *Cell* 71:451-461)) in Drosophila embryos have been showed to act as morphogens *in vitro.* Both of these factors belong to the TGF-β superfamily of peptide growth factors and both can specify different cell fates at tight thresholds of concentration. Since follistatin is an inhibitor of activin and both activin ligand and receptor RNAs are expressed in the presumptive ectoderm, follistatin, like activin, may have morphogenic activity. Both the dominant negative activin receptor and follistatin, as described below, elicit neural tissue formation directly. Based on this data, it is asserted that neural tissue represents the default state vis-a-vis activin signaling in presumptive ectoderm. In this model, the amount of activin ligand and receptor present in the cap maintains the cells as epidermal, and additional activin changes the cells' fate to mesodermal. By inhibiting activin to varying degrees, follistatin could also act as a morphogen.

In an illustrative embodiment of an *in vitro* assay system, to test if follistatin can act as a morphogen, dissociated animal cap cells can be cultured and dosed with activin at a concentration sufficient to turn on a general mesodermal marker such as brachyury upon reassociation. The activity of activin can then be challenged with small incremental changes in follistatin protein concentration. Indicators that follistatin might serve as a morphogen will include the observation of small concentration differences giving rise to different cell fates, distinguishable by histology or through the use of cell-type specific molecular markers. These studies will allow for the determination of the extent of the number of independent cellular fates that exist in the presumptive ectoderm as well as, what proportion of animal cap cells become neural in response to different concentrations of follistatin.

Similar studies can be performed with non-human embryonic stem cells, such as the neural crest cells described above, to determine if the concentration of follistatin is influential on the path of neuronal differentiation of uncommitted and committed progenitor cells, and ultimately whether concentration effects the particular terminally-differentiated derivation of the progenitor cells which arise.

In another embodiment, *in vitro* cell cultures can be used for the identification, isolation, and study of genes and gene products that are expressed in response to disruption of activin signaling, and therefore likely involved in neurogenesis. These genes would be "downstream" of the activin signal, and required for neuronal differentiation. For example, if new transcription is required for the neuralization, a subtractive cDNA library prepared with control animal caps and animal caps treated with follistatin can be used to isolate genes that are turned on or turned off by this process. The powerful subtractive library methodology incorporating PCR technology described by Wang and Brown is an example of a methodology useful in conjunction with the present invention to isolate such genes (Wang et al. (1991) *Proc.Natl.Acad.Sci. USA* 88:11505-11509). For example, this approach has been used successfully to isolate more than sixteen genes involved in tail resorption with and without thyroid hormone treatment in Xenopus. Utilizing control and treated caps, the induced pool can be subtracted from the uninduced pool to isolate genes that are turned on, and then the uninduced pool from the induced pool for genes that are turned off. From this screen, it is expected that two classes of mRNAs can be identified. Class I RNAs would include those RNAs expressed in untreated caps and reduced or eliminated in induced caps, that is the down-regulated population of RNAs. Class II RNAs include RNAs that are upregulated in response to induction and thus more abundant in treated than in untreated caps. RNA extracted from treated vs untreated caps can be used as a primary test for the classification of the clones isolated from the libraries. Clones of each class can be further characterized by sequencing and, their spatiotemporal distribution determined in the embryo by whole mount *in situ* and developmental northern blots analysis.

For example, in one embodiment of this subtractive assay, special attention can be given to genes that prove to be an immediate early response to neural induction. To qualify as such, these genes should fulfill the following four criteria. First, the RNA should appear quickly (10 to 30 minutes) following application of the inducer. To test this requirement, RNA can be isolated at different times from induced caps and scored for gene expression by northern blots. Second, the induction of the gene should not require previous protein synthesis. Thus, caps can be incubated with cycloheximide (5µg/ml) prior to and during short incubation with follistatin (30 minutes) after which the caps can be allowed to remain in follistatin for longer periods of time (90 minutes) and then analyzed by northern blotting. This strategy has been used in a similar situation when Mix. 1, a homeobox gene exhibiting an immediate early response to both activin and bFGF was isolated from Xenopus animal caps (Rosa, F.M. (1989) *Cell.* 57:965-974). These conditions are sufficient to inhibit 75% to 80% of the protein synthesis during the period of induction and to abolish the induction of muscle actin mRNA in response to activin in late explants (Bolce et al. (1993) *Developmental Biology* 160). Third, immediate early response genes should be expressed as a result of contact with the inducer and not from a secondary cell-cell induction. One method to differentiate between these two responses is to dissociate the cells of the animal cap in Ca/Mg free medium, add follistatin and compare the amount of the induced transcript in dissociated cells versus intact caps. If the levels are comparable in both types of caps, then it may be concluded that cell-cell contact was not required for this induction and it is thus likely a direct response of follistatin treatment. Finally, these genes would be expected to be present and activated in the nervous system during neurogenesis.

Once isolated, the genes regulated by follistatin can be sequenced and their non-human embryonic distribution can be determined by wholemount approaches. If their embryonic expression is in agreement with a possible neurogenic function, they can be tested for neuralizing activity in animal caps and in non-human embryos as described herein for follistatin and other NAs.

In addition to the implantation of cells cultured in the presence of follistatin and other *in vitro* uses described above, yet another objective of the present invention concerns the therapeutic application of activin-disrupting agents to enhance survival of neurons and other neuronal cells in both the central nervous system and the peripheral nervous system. The ability of follistatin to regulate neuronal differentiation not only during development of the nervous system but also presumably in the adult state indicates that NAs can be reasonably expected to facilitate control of adult neurons with regard to maintenance, functional performance, and aging of normal cells; repair and regeneration processes in chemically or mechanically lesioned cells; and prevention of degeneration and death which result from loss of differentiation in certain pathological conditions. In light of this understanding, the present invention specifically contemplates applications of follistatin in the preparation of a medicament or the medicaments for the treatment of (prevention and/or reduction of the severity of) neurological conditions deriving from: (i) acute, subacute, or chronic injury to the nervous system, including traumatic injury, chemical injury, vasal injury and deficits (such as the ischemia resulting from stroke), together with infectious/inflammatory and tumor-induced injury; (ii) aging of the nervous system including Alzheimer's disease; (iii) chronic neurodegenerative diseases of the nervous system, including Parkinson's disease, Huntington's chorea, amylotrophic lateral sclerosis and the like, as well as spinocerebellar degenerations; (iv) chronic immunological diseases of the nervous system or affecting the nervous system, including multiple sclerosis; and (v) degenerative diseases of the retina.

Many neurological disorders are associated with degeneration of discrete populations of neuronal elements and may be treated with a therapeutic regimen which includes a neuralizing agent of the present invention. For example, Alzheimer's disease is associated with deficits in several neurotransmitter systems, both those that project to the neocortex and those that reside with the cortex. For instance, the nucleus basalis in patients with Alzheimer's disease were observed to have a profound (75%) loss of neurons compared to age-matched controls. Although Alzheimer's disease is by far the most common form of dementia, several other disorders can produce dementia. Many are age-related, occurring in far greater incidence in older people than in younger. Several of there are degenerative diseases characterized by the death of neurons in various parts of the central nervous system, especially the cerebral cortex. However, some forms of dementia are associated with degeneration of the thalmus or the white matter underlying the cerebral cortex. Here, the cognitive dysfunction results from the isolation of cortical areas by the degeneration of efferents and afferents. Huntington's disease involves the degeneration of intrastraital and cortical cholinergic neurons and GABAergic neurons. Pick's disease is a severe neuronal degeneration in the neocortex of the frontal and anterior temporal lobes, sometimes accompanied by death of neurons in the striatum. Treatment of patients suffering from such degenerative conditions can include the application of medicaments comprising follistatin in order to manipulate, for example, the de-differentiation and apoptosis of neurons which give rise to loss of neurons. In preferred embodiments, a source of follistatin is stereotactically provided within or proximate the area of degeneration.

In addition to degenerative-induced dementias, a pharmaceutical preparation of follistatin can be applied opportunely in the treatment of neurodegenerative disorders which have manifestations of tremors and involuntary movements. Parkinson's disease, for example, primarily affects, subcortical structures and is characterized by degeneration of the nigrostriatal pathway, raphe nuclei, locus cereleus, and the motor nucleus of vagus. Ballism is typically associated with damage to the subthalmic nucleus, often due to acute vascular accident. Also included are neurogenic and myopathic diseases which ultimately affect the somatic division of the peripheral nervous system and are manifest as neuromuscular disorders. Examples include chronic atrophies such as amyotrophic lateral sclerosis, Guillain-Barre syndrome and chronic peripheral neuropathy, as well as other diseases which can be manifest as progressive bulbar palsies or spinal muscular atrophies. The present method is ammenable to the treatment of disorders of the cerebellum which result in hypotonia or ataxia, such as those lesions in the cerebellum which produce disorders in the limbs ipsilateral to the lesion. For instance, a preparation of a neuralizing agent of the present invention can be used to treat a restricted form of cerebellar corical degeneration involving the anterior lobes (vermis and leg areas) such as is common in alcoholic patients.

In yet another embodiment, the subject medicament is used to treat amyotrophic lateral sclerosis. ALS is a name given to a complex of disorders that comprise upper and lower motor neurons. Patients may present with progressive spinal muscular atrophy, progressive bulbar palsy, primary lateral sclerosis, or a combination of these conditions. The major pathological adnomality is characterized by a selective and progressive degeneration of the lower motor neurons in the spinal cord and the upper motor neurons in the cerebral cortex. The therapeutic application of an activin antagonist can be used alone or in conjunction with other neurotrophic factors such as CNTF, BDNF, or NGF to prevent and/or reverse motor neuron degeneration in ALS patients.

Follistatin can also be used in the treatment of autonomic disorders of the peripheral nervous system, which include disorders affecting the innervation of smooth muscle endocrine tissue (such as glandular tissue). For instance, neuralizing agent compositions may be useful to treat tachycardia or atrial cardiac arrythmias which may arise from a degenerative condition of the nerves innervating the striated muscle of the heart.

In yet another embodiment, follistatin can be used in the preparation of a medicament for the treatment of neoplastic or hyperplastic transformations, involving neural tissue. For instance, an activin antagonist likely to be capable of inducing differentiation of transformed neuronal cells to become post-mitotic or possibly apoptotic. Inhibition of activin-mediated inductive events may also involve disruption of autocrine loops, such as PDGF autostimulatory loops, believed to be involved in the neoplastic transformation of several neuronal tumors. The medicament may, therefore, be of use in the treatment of, for example, malignant gliomas, medulloblastomas, neuroectodermal tumors, and ependymonas.

Follistatin or a pharmaceutically acceptable salt thereof, may be conveniently formulated for administration with a biologically acceptable medium, such as water, buffered saline, polyol (for example, glycerol, propylene glycol, liquid polyethylene glycol and the like) or suitable mixtures thereof. The optimum concentration of the active ingredient(s) in the chosen medium can be determined emperically, according to procedures well known to medicinal chemists. As used herein, "biologically acceptable medium" includes any and all solvents, dispersion media, and the like which may be appropriate for the desired route of administration of the pharmaceutical preparation. The use of such media for pharmaceutically active substances is known in the Except insofar as any conventional media or agent is incompatible with the activivity follistatin, its use in the pharamceutical preparation of the invention is contemplated. Suitable vehicles and their formulation inclusive of other proteins are described, for example, in the book *Remington's Pharmaceutical Sciences* (Remington's Pharmaceutical Sciences. Mack Publishing Company, Easton, Pa., USA 1985), These vehicles include injectable "deposit formulations". Based on the above, the pharmaceutical formulation includes, although not exclusively, NA solutions or a freeze-dried powder of follistatin in association with one or more pharmaceutically acceptable vehicles or diluents, and contained in buffered media at a suitable pH and isosmotic with physiological fluids. For illustrative purposes only and without being limited by the same, possible composition of formulations which may be prepared in the form of solutions for the treatment of nervous sytem disorders with follistatin are given in the della Valle U.S. Patent No. 5,218,094. In the case of freeze-dried preparations, supporting excipients such as, but not exclusively, mannitol or glycine may be used and appropriate buffered solutions of the desired volume will be provided so as to obtain adequate isotonic buffered solutions of the desired pH. Similar solutions may also be used for the pharmaceutical compositions of follistatin in isotonic solutions of the desired volume and include, but not exclusively, the use of buffered saline solutions with phosphate or citrate at suitable concentrations so as to obtain at all times isotonic pharmaceutical preparations of the desired pH, for example, neutral pH.

Methods of introduction of follistatin at the site of treatment include, but are not limited to, intradermal, intramuscular, intraperitoneal, intravenous, subcutaneous, oral, and intranasal. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir.

Methods of introduction may also be provided by rechargable or biodegradable devices. Various slow release polymeric devices have been developed and tested *in vivo* in recent years for the controlled delivery of drugs, including proteinacious biopharmaceuticals. A variety of biocompatible polymers (including hydrogels), including both biodegradable and non-degradable polymers, can be used to form an implant for the sustained release of follistatin at a particular target site. Such embodiments of the present invention can be used for the delivery of an exogenously purified follistatin, which has been incorporated in the polymeric device, or for the delivery of follistatin produced by a cell encapsulated in the polymeric device.

An essential feature of certain embodiments of the implant is the linear release of the NA, which can be achieved through the manipulation of the polmer composition and form. By choice of monomer composition or polymerization technique, the amount of water, porosity and consequent permeability characteristics can be controlled. The selection of the shape, size, polymer, and method for implantation can be determined on an individual basis according to the disorder to be treated and the individual patient response. The generation of such implants is generally known in the art. See, for example, *Concise Encylopedia of Medical & Dental Materials,* ed. by David Williams (MIT Press: Cambridge, MA, 1990); and the Sabel et al. U.S. Patent No. 4,883,666. In another embodiment of an implant, a source of cells producing the NA, or a solution of hydogel matrix containing purifed NA, is encapsulated in implantable hollow fibers. Such fibers can be pre-spun and subsequently loaded with the NA source (Aebischer et al. U.S. Patent No. 4,892,538; Aebischer et al. U.S. Patent No. 5,106,627; Hoffman et al. (1990) *Expt. Neurobiol.* 110:39-44; Jaeger et al. (1990) *Prog. Brain Res.* 82:41-46; and Aebischer et al. (1991) *J. Biomech. Eng.* 113:178-183), or can be co-extruded with a polymer which acts to form a polymeric coat about the NA source (Lim U.S. Patent No. 4,391,909; Sefton U.S. Patent No. 4,353,888; Sugamori et al. (1989) *Trans. Am. Artif. Intern. Organs* 35:791-799; Sefton et al. (1987) *Biotehnol. Bioeng.* 29:1135-1143; and Aebischer et al. (1991) *Biomaterials* 12:50-55).

In yet another embodiment of the present invention, follistatin can be administered as part of a combinatorial therapy with other agents. For example, the combinatorial therapy or medicament can include follistatin with at least one trophic factor. Exemplary trophic factors include nerve growth factor, cilliary neurotrophic growth factor, schwanoma-derived growth factor, glial growth factor, stiatal-derived neuronotrophic factor, platelet-derived growth factor, and scatter factor (HGF-SF). Other neural inductive proteins, such as *hedgehog*-like proteins, *noggin,* and ligands of the *Notch* receptor may also be used in conjunction with the subject neuralizing agent. Antimitogenic agents can also be used, as for example, when proliferation of surrounding glial cells or astrocytes is undesirable in the regeneration of nerve cells. Examples of such antimitotic agents include cytosine, arabinoside, 5-fluorouracil, hydrozyurea, and methotrexate.

### Exemplification

The fact that neuralization was closely linked to mesoderm induction has hampered most of the previous effort invested in the molecular characterization of neural inducers and patterners. Thus, attempts to isolate factors involved in neural induction and patterning have ended in the identification and characterization of mesoderm inducers and modifiers. The data described in the Examples below demonstrate that the activin antagonist, follistatin both elicits direct neuralization in embryonic explants without a prerequirement for mesoderm induction. In addition, a full length cDNA for Xenopus follistatin has been isolated and its embryonic localization shown to be in perfect agreement with a role in neural development *in vivo.* The observation that antagonizing the activin signal results in neuralization suggests, for the first time, that neural induction in vertebrates represents a default state.

The invention now being generally described, it will be more readily understood by reference to the following examples which are included merely for purposes of illustration of certain aspects and embodiments of the present invention, and are not intended to limit the invention.

### Example 8.

### Cloning of the Xenopus follistatin

To further address whether activin is the endogenous inhibitor of neural differentiation, the activity of two known specific inhibitors of activin follistatin and inhibin, were assayed. Since no Xenopus full length cDNAs for these two proteins were available, the possible neuralizing activity of the corresponding rat genes were originally tested *in vitro* by the animal cap assay. Neuralization of Xenopus embryonic tissue was observed with both rat follistatin and inhibin.

The same experiments were also performed with the Xenopus homologs. To perform this experiment, a full length follistatin clone from a Xenopus cDNA library was isolated by standard protocols. The sequence of this clone contains a signal peptide, which is typically indicative of a secreted factor, and three closely related domains, previously reported as "follistatin modules" (Patthy et al. (1993) *Trends in Neurol. Science* 16:76-81), that encompass about 75% of the mature protein. It is noted that follistatin modules have been recently recognized in at least four other proteins such as osteonectin, agrin, a protein SC1 from rat brain, and human testican. While SC1, testican and osteonectin have one follistatin module, agrin has nine tandemly repeated modules at its N-terminus (Patthy et al. (1993) *Trends in Neurol. Science* 16:76-81).

In all species from which the follistatin cDNAs have been isolated, including humans, pigs, rats and Xenopus, two type of transcripts have been detected. Sequence analysis, S1 mapping and RNase protection using the human, porcine and rat genomic clones have revealed that these two transcripts are generated by differential splicing. This differential splicing leads to the translation of two monomeric glycosylated proteins. The smaller molecular weight form in all species studied so far represents a carboxy-truncated form of the larger precursor and is generated by the removal of a highly acidic stretch of acidic amino acids from the larger precursor. The number of amino acids in each protein is used conventionally to name each subtype. Thus the human follistatin proteins are called FS 315 and FS 288; the rat, FS 344 and FS 317; and, the pig, FS 300 and FS 288. Using the same convention, the Xenopus follistatin is termed XFS 319. This protein is the homolog of the smaller subtype of follistatin cloned from other species and lacks the acidic carboxy terminus. Beside the two different lengths of the FS amino acid chains, the native proteins show variations in their degree of glycosylation, which also contributes to the heterogeneity in molecular weight of follistatin (Inouye et al. (1991) *Endocrinology.* 129:815-822).

### Example 9

### Generation of follistatin protein, synthetic RNA and expression vectors

A plasmid allowing the production of large amount of active synthetic DNA for follistatin was created. This plasmid pSP64TXFS-319 was generated by subcloning the open reading frame of XFS-319 (SEQ ID No. 1) into the plasmid pSP64T. pSP64T is a derivative of the publicly available pSP64 vector which has been altered to include 5' and 3' untranslated regions of the xenopus β-globin gene to enhance mRNA stability and translation. This construct allows the production of large quantities of capped RNAs that is stable and translationaly enhanced. An epitope tagged version of XFS-319, that is as active as the wild type construct in blocking activin, was generated. This was constructed by adding 12 amino acids originally derived from the human myc protein to the C-terminal end of XFS-319. This molecular tag is recognized by a monoclonal antibody (Ma19E10) and allows the injected follistatin protein to be followed in whole embryos.

Cell culture lines which overexpress a Xenopus follistatin (XFS-319) can be constructed using the pSP64TXFS-319 construct. Following a protocol that has been proven successful in overexpressing human follistatins in Chinese Hamster Ovary cell line (CHO cells) (Inouye et al. (1991) *Endocrinology.* 129:815-822), the open reading frame of XFS-319 can be subcloned into a plasmid containing an SV40 promoter and polyadenylation sequence (pSV2). This vector along with the same vector expressing dhfr (pSV2dhfr) is co-transfected by the calcium phosphate precipitation method into a dhfr-deficient CHO cell line (CHO-DG44). The Xenopus gene is amplified using methotrexate (MTX). Conditioned medium from these transfected cells, along with the untransfected controls, can be used directly, or, where purified protein is required, an activin affinity column can be employed to purify XFS-319 from the conditioned medium as previously described (Inouye et al. (1991) *Endocrinology.* 129:815-822).

Several types of expression vectors can be used for the expression of XFS-319 in embryos. For example, XFS-319 can be placed under the control of the cytoskeletal actin promoter; a powerful, constitutively active promoter. Similarly, XFS-319 can be placed under the control of the Xenopus heat shock promoter. This promoter is only active when the temperature of the recipient embryos, usually kept at 20°C, is raised to 24°C- 25°C (Harland et al. (1988) *Development* 102:837-852; and Vize et al. in *Xenopus laevis:* Practical uses in Cell and Molecular Biology.ed. Kay and Peng, Academic Press Inc:Florida, 1991). As zygotic transcription in Xenopus does not begin until mid-blastula stages (MBT) such constructs can be useful in determining the later function of follistatin in embryos.

### Example 10

### Expression of follistatin during Xenopus development

Northern blot analysis of RNA from different embryonic stages revealed that, as it is the case in mammals, two distinct RNAs of 2.4 and 3.6 kb are transcribed from the follistatin gene, and that the 2.4 kb message is present maternally in the fertilized egg. A partial cDNA clone encoding part of the larger Xenopus follistatin subtype was also isolated recently from Xenopus embryos (Tashiro et al. (1991) *Biochem Biophys Res Comm.* 174:1022-1027). The amino acid identity among species is extremely high, among mammals there is about 98% identity (Inouye et al. (1991) *Endocrinology.* 129:815-822) and between Xenopus and humans there is 86% identity.

The differential splicing leading to the generation of the two protein types does not seem to be under tissue specific regulation since both transcripts are present in all tissues expressing follistatin. The shorter of the human follistatin (HFS 288) has been shown to be 8-10 times more potent in inhibiting activin both *in vitro* and *in vivo* (Inouye et al. (1991) *Endocrinology.* 129:815-822). In fact, the shorter form of the human follistatin is the most potent inhibitor of activin, even better than inhibin, the other specific inhibitor of activin (Inouye et al. (1991) *Endocrinology.* 129:815-822). Kinetic analyses of a mixture of human FS binding to activin using a solid phase assay revealed that the FS-activin interaction is of high affinity similar to that estimated for activin binding to its receptor. Inhibin is approximately 500-1000-fold lower in relative potency as compared to activin in the FS binding assay.

### Example 11

### Follistatin is localized in the Spemann organizer

Whole mount *in situ* hybridization (Hemmati-Brivanlou et al. (1990) *Development* 110:325-330) with an anti-sense XFS-319 RNA was used to detect the spatial distribution of follistatin during different stages of Xenopus embryogenesis. The probe used in these experiments is that used on the northern blot and thus can recognize both follistatin transcripts. By this approach, follistatin RNA was first detected at the onset of gastrulation where a few cells of the organizer express XFS transcripts. In the gastrula, follistatin RNA is localized to the dorsal side. The localization is confirmed by RT-PCR on RNAs extracted from disected embryos at the onset of gastrulation, using the dorsally localized markers *noggin* (Smith et al. (1992) *Cell* 70:829-840) and Goosecoid (Blumberg et al. (1991) *Science 253:194-196)* as controls.

This region of the embryo has previously been characterized as a potent neural inducer in the animal cap assay. Expressions continues in the anterior two-thirds of the notochord in late gastrula and early neurula stage embryos. Transverse sections of these embryonic stages show that follistatin RNA is expressed in a subset of cells of the prechordal and chordal mesoderm. This expression is strongest in the anterior involuting mesoderm and fades in intensity toward the posterior end. In early neurulae, when the neural plate is still open, the expression of follistatin is confined to head mesoderm and anterior notochord. A traverse section of these early neurula show that, in addition to the expression in the dorsal mesoderm, follistatin RNA can also be detected in a few cells of the hypochord, just ventral to the notochord. At these stages, the portion of the anterior notochord expressing follistatin touches the floor of the diencephalon (forebrain) and underlies the midbrain, hindbrain, and about half of the anterior spinal chord. The expression in the notochord extends posteriorly as development procedes and includes the entire notochord by stage 21. The organizer region of the embryo has previously been characterized as a potent neural inducer in the animal cap assay. Thus, the temporal and spatial localization of follistatin is in perfect agreement with the site generally predicted to contain the neural inducing activity.

After the neural tube is formed, follistatin RNA is also detected in the forebrain, presumptive midbrain, midbrain-hindbrain junction, and hindbrain. Furthermore, there is a transient follistatin expression in the pronephrous and ventrolateral mesoderm near the blood islands. There is also expression in photoreceptor of the retina at the tailbud stage. At the swimming tadpole stage, most of the expression is localized to the notochord and the head. In the forebrain, the expression of follistatin is confined to three stripes distributed dorsoventrally in the cells of the ventricular zone. There is no apparent staining in the floor or roofplate of the neural tube.

### Example 12

### Xenopus Follistatin RNA and Protein Block Mesoderm Formation and Morphogenetic Movements Induced by Activin

The ability of follistatin to inhibit activin activity was tested by two independent approaches. In the first set of experiments, embryos were injected at the 2-cell stage in the animal pole of both blastomeres with 1 ng of either XFS-319 RNA or globin control RNA. The embryos were allowed to develop until they reached blastula stage 8, at which point the animal caps were dissected and incubated in either buffer alone or activin. While injection of control globin RNA apparently had no effect on activin induced morphogenetic movements, injection of XFS-319 completely blocked the morphogenetic movements associated with induction by activin treatment. In addition, animal caps injected with XFS-319 and incubated in buffer or activin were found to show clear cement glands, which parallels the effect of the dominant negative activin receptor described above.

In the second set of experiments, either 50 ng of RNA encoding XFS-319 or 12 ng of RNA encoding Xenopus activin β_{b} was injected into mature (stage 6) Xenopus oocytes. After 48 hr, medium conditioned by these oocytes or by uninjected oocytes was collected and applied to animal cap explants of stage 8 blastula embryos. It was observed that when the explants were incubated in either buffer alone or conditioned medium from uninjected oocytes, they remained spherical. In contrast, explants incubated in conditioned medium from activin-injected oocytes elongate drastically. Animal caps incubated in medium conditioned by oocytes injected with XFS-319 remained spherical. Finally, when conditioned media from oocytes injected with activin and XFS-319 were mixed at 1:1 ratio, the morphogenetic movement induced by activin is completely blocked. The results of these two experiments demonstrate that XFS-319, like its homologs in other species, is a potent inhibitor of activin.

To assess the potency of this inhibition, a dose response study was carried out in which the animal poles of 2-cell stage embryos were injected with either control globin RNA or different concentrations of XFS-319. As in the first set of experiments, animal caps from injected embryos were explanted at stage 8 and incubated with the potent mesoderm inducer activin. These caps were cultured until sibling uninjected embryos reached tailbud stage, at which point total RNA was isolated from the explants and analyzed by Northern blotting. While 4 ng of the control globin RNA did not seem to interfere with the mesoderm inducing activity of activin, as assayed by the expression of the axial mesodermal marker muscle actin, 250 pg of XFS-319 RNA was found to be enough to block activin action completely. In addition, none of the animal caps injected with 4.00, 1.00, or 0.25 ng of XFS-319 RNA and treated with activin (20 explants for each concentration) showed any sign of elongation.

### Example 13

### Follistatin Is a Direct Neural Inducer

We tested for direct neural inducing activity of XFS-319 was also tested in animal cap explants. An indication that XFS-319 might possess this type of activity came from the observation that cement glands were induced in explants injected with XFS-319. Embryos were injected at the 2-cell stage in the animal pole with 2 ng of XFS-319 RNA, and it was found that the injection of this RNA elicits induction of neural tissue in the animal cap, as demonstrated by the expression of the general neural markers N-CAM and β-tubulin isotype II. These markers were expressed in the absence of muscle actin, suggesting that this induction is direct, i.e., without concomitant mesoderm induction. It is further noted that this represents the first evidence of a single endogenous embryonic molecule with neural inducing folllistation also provides evidence for activin as the inhibitor of neuralization.

Induction of the general neural marker in these explants clearly demonstrates the neuralizing activity of XFS-319. However, although this induction happens in the absence of the muscle actin marker, other mesodermal tissues could still be present. To address this question, XFS-319 or a control RNA was injected under the same conditions as described above. A portion of the animal cap explants were processed when sibling controls reached midgastrula stage to analyze the expression of immediate early mesodermal markers, and the rest were allowed to develop until tailbud stage and then assayed for muscle actin and neural markers. Uninjected animal caps, or explants injected with either XFS-319 or control RNA incubated in buffer alone, failed to express any of the five mesodermal markers assayed. The early dorsal-specific mesodermal markers goosecoid and noggin, as well as the ventral marker Xwnt-8(Christian et al. (1991) Development 111:1045-1056 and the general early mesodermal markers X-bra (Smith et al., (1991) Cell 67:79-87) and Mix-1 are not expressed in caps that were injected with 2 ng of XFS-319 RNA. Uninjected animal caps, in contrast, did express all of these markers in reponse to activin. In addition, the explants that were left to develop until tailbud stage also were found to express the neural marker N-CAM, but not muscle actin, demonstrating that XFS-319 was active in inducing neural tissue in these experiments. These experiments further support the notion that the neuralizing activity of XFS-319 happens in the absence of scorable mesoderm and is, by this definition, direct. In addition, the fact that follistatin does not induce noggin expression suggests that either the mechanism of neural induction by follistatin is independent of noggin action or that follistatin acts downstream of noggin in this regard.

### Example 14

### Dose of Folliststin RNA Required for Neural Induction

We next aimed to characterize the direct neural inducing activity of follistatin by measuring the doses required for induction of a general neural marker and cement glands in animal cap explants. Embryos were injected at the 2-cell stage in the animal pole with different concentrations of XFS-319 RNA. Animal caps from injected embryos were explanted at the blastula stage and allowed to develop until sibling uninjected controls reached the early tailbud stage. RNA extracted from explants injected with different concentrations of XFS-319 along with RNA from uninjected explants and control embryos were analyzed by Northern blots. It was also observed that the general neural marker β-tubulin isotype II can be induced in these caps by injection of as little as 50 pg of XFS-319 RNA, while the cement gland markerXAG-1 (Sive et al., (1989) Cell 58:171-180) requires a minimum of 250 pg. The muscle actin panel again demonstrated that neuralization was direct and that the explants did not contain mesoderm.

### Example 15

### Follistatin Induces Anterior Neural Markers

Examples 1-7 above demonstrate that interference with signaling through the type II activin receptor in embryonic explants results in the induction of anterior neural markers. Since follistatin interferes with activin signaling by a different mechanism, it is important to ask what type of neural tissue is induced by XFS-319 expression in animal cap explants. To this end, the same experiment described in Example 13 was performed, and RT-PCR was used to score for the range of anteroposterior neural markers. As was the case for the truncated activin receptor, the anterior neural markers opsin, which demarcates the photoreceptors of the retina derived from the forebrain, En-2, a marker of the midbrain-hindbrain junction, and tanabin, principally a marker of hindbrain, trigeminal ganglia, and a few cells of the eye, forebrain, and spinal cord were all induced by follistatin. Interestingly, Krox-20, another hindbrain marker, and Xlhbox-6, the spinal cord marker, are not detected in these caps. These results are summarized in Table 3.

**TABLE 3**

| *Neural Markers Scored in Animal Caps Injected with XFS-319 RNA* | |
|---|---|
| Marker | Expression |
| General Neural Matters | |
| N-CAM | + |
| β-Tubulin Isotype II | + |
| Anteroposterior Markers | |
| Opsin | + |
| Tanabin | + |
| *En-2* | + |
| *Krox-20* | - |
| *Xlhbox-6* | - |
| Dorsoventral Marker | |
| Other Ectodermal Marker | |
| *XAG1* | + |

For Table 3: markers in all cases have been scored when sibling controls have reached the tailbud stage. N-CAM and β-tubulin isotype II are general neural markers and are both expressed. The anteroposterior markers include opsin, which is a marker of the photoreceptors of the eye, *En-2,* which demarcates posterior midbrain and anterior hindbrain, *Krox-20,* which demarcates rhombomeres 3 and 5 of the hindbrain, and tanabin, a marker of a few cells of the forebrain and retina but mostly rhombomeres 2, 4, 6, and 8 and the trigeminal ganglia. *Xlhbox-6* is a marker of the spinal cord. *XAG1* is a cement gland marker. The data presented in this table are a combination of results obtained by Northern blot and RT-PCR.

From these results, we conclude that anterior neural tissue such as forebrain and midbrain is present in animal caps expressing XFS-319. The presence of hindbrain, however, is not well established by this type of assay. The absence of Krox-20 expression suggests that rhombomeres 3 and 5 are not present in these explants. Tanabin expression can be interpreted either as the presence of even-numbered rhombomeres or simply as confirmation of the presence of midbrain or forebrain. The absence of Xlhbox-6 signal suggests that posterior neural tissue such as spinal cord is absent. The staining patterns for N-CAM and muscle actin confirmed that this neuralization is direct.

### Example 16

### Actinin, but Not Noggin, Induces the Expression of Follistatin

The protein noggin has been previously demonstrated to induce neural tissue in animal cap explants (Lamb et al., (1993) Science 262:713-718). However, while neural induction by noggin is direct, activin, in contrast, is not apparently a direct neural inducer, and the neural tissue in explants treated with activin likely results from a secondary induction by mesoderm. It has been previously shown (Thomsen and Melton, (1993) Cell 74:433-441)), and we have confirmed, that activin can induce the expression of noggin in animal cap explants (see Example 13). Thus, it could be argued that the indirect neural inducing activity of activin is mediated by noggin. Follistatin displays direct neural inducing activity in explants; however, Example 13 demonstrates that this neural induction is not mediated by noggin. To investigate further a possible relationship among the neural inducing activity of noggin, follistatin, and activin, we decided to test the following: first, whether the neural inducing activity of noggin resulted in follistatin expression, and second, whether the neural induction in explants and embryos, in response to activin, could be correlated with follistatin expression.

Embryos at the 2-cell stage were injected with 1 ng of noggin RNA in the animal pole. At blastula stage (stage 8), the animal poles of these embryos along with those of the control injected and uninjected embryos were explanted. Half of the explants were allowed to develop until mid-gastrula stage (stage 10.5), when neural induction has begun, and then were assayed by RT-PCR for follistatin expression. The other half were allowed to develop until early tailbud stage to provide a position control for the function of noggin. Under this experimental condition, the expression of noggin in animal cap explants was not observed to induce follistatin. Thus, the neural inducing activity of noggin in animal cap explants is not mediated, at least at the transcriptional level, by follistatin. Noggin in these experiments has apparently been active in inducing the neural marker N-CAM directly in the explants.

To determine if addition of activin to animal cap explants induces the expression of follistatin, uninjected animal caps dissected at stage 8 were incubated in buffer alone or in the presence of activin. We found that addition of activin to animal caps induces the expression of XFS-319 RNA. Since activin can also induce the expression of noggin in these explants, we conclude that the neural inducing activity of activin may be mediated through either noggin or follistatin, or both.

### Example 17

### Follistatin Expression in Secondary Axes Induced by Activin

When injected into the ventral side of the embryo, activin RNA can also induce a partial secondary axis that includes spinal cord and hindbrain, but not more anterior structures (Thomsen et al., (1990) Cell 63:485-493). Lineage-tracing experiments have shown that the cells that have received activin RNA do not participate in the induced ectopic neural tissue. Thus, we were prompted to ask whether follistatin could be involved in the induction of the ectopic neuraxis. Xenopus activin β_{b} RNA or control RNA was injected into a single blastomere on the ventral side of embryos at the 8- to 16-cell stage. Embryos injected with Xenopus activin β_{b} transcript, but not control RNA, displayed a partial secondary dorsal axis, as previously reported. These embryos were analyzed at neurula stages (stage 21) for the expression and distribution of follistatin RNA by whole-mount in situ hybridization. It was noted that while follistatin RNA is present in the single notochord of the control, most embryos with double axes show the presence of follistatin in both the primary and the secondary axis (n = 18 of 25). Thus, the presence of follistatin in the secondary axis may account for the induction of the secondary neuraxis. These experiments demonstrate that activin can induce the expression of follistatin both in embryonic explants and in the context of the whole embryo. Thus, the neural inducing activity of activin may be mediated by either noggin or follistatin, or both.

### Example 18

### Ectopic injection of follistatin in wild type embryos

There is a direct correlation between the competence of the ectodermal cells to respond to a neural inductive stimulus and the size of the neural plate. To assay follistatin's effect on the competence of the ectoderm, follistatin RNA can be injected in the animal pole of either one or both cells of a two cell stage embryo. For example, experimental embryos can be coinjected with different concentrations of myc-tagged follistatin and a single concentration of β-Gal. Control embryos will be injected with β-Gal alone. The embryos are allowed to develop until they reach the midneurula stage when the neural plate is still open, and then stained as wholemounts for β-Gal and NCAM simultaneously. Since the animal pole of the embryo contributes mostly to epidermal and neural derivatives, the control embryos are expected to display normal size neural plates with β-Gal staining in both the neuroectoderm and the epidermis. Several possible outcomes exist for the experimental embryos. In one scenario, no changes will be detected in the size of the neural plate regardless of the β-Gal distribution, indicating that follistatin did not modify the competence of the animal cap. In another scenario, the embryos still have an expanded neural plate and ectopic neural tissue, with most of the β-Gal and myc positive cells localized within the expanded neural tissue. This latter result would imply that follistatin is capable of changing the competence of the ectodermal cells for neural induction and has led to recruitment of epidermal-fated cells to adopt a neural fate.

The ability of follistatin to induce neural structures on the ventral side can also be tested in similar fashion in the context of the whole embryo. To this end, follistatin and β-Gal RNA, or β-Gal RNA alone, is injected in the ventral side of an 8 cell stage embryo. Control and experimental embryos are harvested when sibling controls reach the tailbud stage, and examined by morphology, histology and wholemount staining. Staining for NCAM can also be used to detect any ectopic neural tissue. The formation of a secondary neuroaxis or neural tissue would clearly indicate that follistatin can neuralize *in vivo.* The β-Gal staining would again be informative as to the fate of the cells making the follistatin protein. In Spemann's original experiment, most of the neural tissue in the secondary axis was derived from the ventral side of the host embryo and did not originate from the organizer. If ,β-Gal expressing cells do not participate in the induced ectopic neuroaxis, then experiment have demonstrated that follistatin neural inducing ability parallels the neural inducer of the organizer. If no ectopic neural tissue is generated, then it might be concluded that other factors from the organizer are also required for the ectopic neural induction. Finally if all cells in the secondary neuroaxis have β-Gal, it would be consistent with a conclusion that even though follistatin can neuralize, this neuralization is not the same as the pathway used by the embryo.

### Example 19

### Injection of Follistatin in UV embryo

A more stringent assay of the neuralizing activity of follistatin *in vivo* is to test the ability of this factor to induce a neuroaxis in embryos completely lacking dorsal structures. UV irradiation of the Xenopus embryo during the first cell-cycle leads to embryos lacking a dorsal axis. These embryos have previously been used in an assay system to isolate factors involved in induction and patterning of the axial mesoderm (Smith et al. (1992) *Cell* 70:829-840). In addition most growth factors with organizer activity can rescue a complete or partial dorsal axis in these ventralized embryo. In one embodiment of an assay for other neuralizing activities, β-Gal and different concentrations of follistatin RNA are co-injected into a single blastomere of UV irradiated embryos. The embryos are then allowed to develop until early tailbud stages at which point they are examined by histology and stained as whole mount for β-Gal and NCAM protein. The control embryos can comprise β-Gal injected alone and uninjected embryos. The two latter controls allow assessment of the quality of UV ventralization. A given concentration of follistatin either will or will not induce an ectopic neural tissue. If it does, the level of β-Gal positive cells populating the induced neuroaxis can be determined. The character of the mesoderm surrounding the neural tissue can also be assessed to see if it maintains its ventral nature or whether it has been dorsalized. If the mesoderm is still ventral in nature, int might be concluded that follistatin has recruited ventral ectodermal cells for the formation of the ectopic neural tissue. If the mesoderm has been dorsalized (i.e. if markers of dorsal mesoderm such as muscle actin or notochord are present) then whether the mesodermal or the neural tissue was induced first will need to be determined.

## Claims

1. A method for inducing a stem cell to differentiate to a neuronal cell phenotype *in vitro,* comprising contacting said cell with an agent that antagonizes the biological action of activin, said activin normally inducing said cell to differentiate to a non-neuronal phenotype, wherein said agent is a follistatin protein and wherein said stem cell is other than a human embryonic cell.

2. The method of claim 1, wherein said agent inhibits the biological activity of activin by preventing said activin from binding activin receptors on the surface of said cell.

3. The method of claim 2, wherein said agent binds activin and sequesters activin such that it cannot bind said activin receptors.

4. The method of claim 1. wherein the cell is a human cell.

5. The method of claim 2, wherein said agent inhibits binding of activin with activin receptors via its own binding to said activin receptors.

6. The method of claim 1, wherein said cell is further contacted with a second growth factor having neurotrophic or neural inductive activity, such as a nerve growth factor, cilliary neurotrophic growth factor, schwanoma-derived growth factor, glial growth factor, stiatal-derived neuronotrophic factor, platelet-derived growth factor, scatter factor, a vertebrate *hedgehog* protein, noggin, and a ligand for a *Notch* receptor.

7. The method of claim 1, wherein said cell is a neural progenitor cell.

8. The method of claim 1, wherein said cell is selected from a melanocyte progenitor cell, a glial progenitor cell, a sensory neuron progenitor cell, a sympatho-adrenal progenitor cell, a parasympathetic progenitor cell, and an enterie progenitor cell.

9. The method of claim 1, wherein said cell is selected from an embryonic cell, a fetal cell, and a neonatal cell, and wherein said embryonic cell is a non-human embryonic cell.

10. A method fur preventing death of a neuronal progenitor cell *in vitro,* comprising contacting said cell with an agent that antagonizes the biological action of activin, said activin normally inducing said cell to differentiate to a non-neuronal phenotype, wherein said agent is a follistatin protein and wherein said method does not involve the use of human embryonic cells.

11. The method of claim 10, wherein said cell is further contacted with a second growth factor having neurotrophic activity, such as a nerve growth factor, cilliary neurotrophic growth factor, schwanoma-derived growth factor, glial growth factor, striatal-derived neuronotrophic factor, platelet-derived growth factor, scatter factor, a vertebrate *hedgehog* protein, noggin, and a ligand for a *Notch* receptor.

12. The method of claim 10, wherein the neuronal progenitor cell is a human cell.

13. A method for inducing a progenitor cell to differentiate along a determined neuronal pathway *in vitro,* comprising contacting said cell with an agent that disrupts an activin signaling pathway in said cell, said signaling pathway normally inducing said cell to differentiate to a non-neuronal cell-type, wherein said agent is a follistatin protein and wherein said method does not involve the use of human embryonic cells.

14. A kit for inducing a normal, non-neoplastic cell to differentiate along a determined neuronal pathway, comprising
(i) a pharmaceutical formulation of an agent that antagonizes activin signaling in said cell, wherein said agent is a follistatin protein, and
(ii) a pharmaceutical formulation of a growth factor having neurotrophic or neural inductive activity.
wherein said agent and growth factor are provided in amounts sufficient to induce or maintain differentiation of said cell to a neuronal cell phenotype.

15. The kit of claim 14, wherein said growth factor is selected from a nerve growth factor, cilliary neurotrophic growth factor, schwanoma-dcrivcd growth factor, glial growth factor, striatal-derived neuronotrophic factor, platelet-derived growth factor, scatter factor, a vertebrate *hedgehog* protein, noggin, and a ligand for a *Notch* receptor.

16. The kit of claim 14, wherein said agent inhibits binding of activin with activin receptors via its own binding to said activin receptors.

17. Use of an agent that antagonizes activin-induced signal transduction in the manufacture of a medicament for effecting, *in vivo,* maintenance, functional performance, and aging of normal neuronal cells of an adult patient, and repair and regeneration of chemically or mechanically lesioned neuronal cells, wherein said agent is a follistatin protein.

18. Use of a therapeutically effective amount of an agent that antagonizes the biological action of activin for the preparation of a medicament for treating a degenerative disorder of the nervous system **characterized by** neuronal cell death in an adult patient, said activin normally inducing cells in said patient to differentiate to a non-neuronal phenotype, wherein said agent is a follistatin protein.

19. The use of claim 18, wherein said therapeutically effective amount of said agent inhibits the de-differentiation of neuronal cells of said patient.

20. The use of claim 18, wherein said therapeutically effective amount of said agent induces the terminal differentiation of cells of said patient to a neural cell phenotype.

21. The use of claim 20, wherein said neural cell phenotype is a glial cell.

22. The use of claim 20 wherein said neural cell phenotype is a nerve cell.

23. The use of claim 18 wherein said degenerative disorder is a neuromuscular disorder.

24. The use of claim 18, wherein said degenerative disorder is an autonomic disorder.

25. The use of claim 18, wherein said degenerative disorder is a central nervous system disorder.

26. The use of claim 18, wherein said degenerative disorder is selected from Alzheimer's disease, Parkinson's disease, amyotrophic lateral sclerosis, Pick's disease, Huntington's disease, multiple sclerosis, neuronal damage resulting from anoxia-ischemia, neuronal damage resulting from trauma, and neuronal degeneration associated with a natural aging process.

27. The use of claim 18, wherein said medicament further includes a therapeutically effective amount of a second growth factor having neurotrophic activity.

28. The use of claim 27, wherein said second growth factor is selected from a nerve growth factor, cilliary neurotrophic growth factor, schwanoma-derived growth factor, glial growth factor, striatal-derived neuronotrophic factor, platelet-derived growth factor.

29. The use of claim 18, wherein said medicament further includes a therapeutically effective amount of an antimitotic agent.

30. The use of claim 29, wherein said antimitotic agent is selected from cytosine, arabinoside, 5-fluorouracil, hydroxyurea, and methotrexate.

31. The use of claim 18, wherein the medicament is formulated for administration to a human.

## Patentansprüche

1. Verfahren zum Induzieren einer Stammzelle, *in vitro* zu einem Phänotyp einer neuronalen Zelle zu differenzieren, umfassend Inkontaktbringen der Zelle mit einem Mittel, das die biologische Wirkung von Activin antagonisiert, wobei das Activin normalerweise die Zelle induziert, zu einem nicht-neuronalen Phänotyp zu differenzieren, wobei das Mittel ein Follistatin-Protein ist und wobei die Stammzelle anders als eine humane embryonale Zelle ist.

2. Verfahren nach Anspruch 1, wobei das Mittel die biologische Aktivität von Activin hemmt, indem es das Activin am Binden von Activin-Rezeptoren auf der Oberfläche der Zelle hindert.

3. Verfahren nach Anspruch 2, wobei das Mittel Activin bindet und Activin sequestriert, derart, daß es die Activin-Rezeptoren nicht binden kann.

4. Verfahren nach Anspruch 1, wobei die Zelle eine humane Zelle ist.

5. Verfahren nach Anspruch 2, wobei das Mittel die Bindung von Activin mit Activin-Rezeptoren durch seine eigene Bindung an die Activin-Rezeptoren hemmt.

6. Verfahren nach Anspruch 1, wobei die Zelle weiter mit einem zweiten Wachstumsfaktor mit neurotropher oder neuraler induktiver Aktivität, wie beispielsweise ein Nervenwachstumsfaktor, ziliarer neurotropher Wachstumsfaktor, Schwannomabgeleiteter Wachstumsfaktor, glialer Wachstumsfaktor, striatal abgeleiteter neuronotropher Faktor, Plättchen-abgeleiteter Wachstumsfaktor, Streufaktor, ein Wirbeltier-*Hedgehog*-Protein, Noggin und ein Ligand für einen Notch-Rezeptor, in Kontakt gebracht wird.

7. Verfahren nach Anspruch 1, wobei die Zelle eine neurale Vorläuferzelle ist.

8. Verfahren nach Anspruch 1, wobei die Zelle aus einer Melanozyten-Vorläuferzelle, einer Glia-Vorläuferzelle, einer Sinnesneuronen-Vorläuferzelle, einer sympatho-adrenalen Vorläuferzelle, einer parasympathischen Vorläuferzelle und einer enterischen Vorläuferzelle ausgewählt ist.

9. Verfahren nach Anspruch 1, wobei die Zelle aus einer embryonalen Zelle, einer fetalen Zelle und einer neonatalen Zelle ausgewählt ist und wobei die embryonale Zelle eine nicht-humane embryonale Zelle ist.

10. Verfahren zum Verhindern des Todes einer neuronalen Vorläuferzelle *in vitro,* umfassend Inkontaktbringen der Zelle mit einem Mittel, das die biologische Wirkung von Activin antagonisiert, wobei das Activin normalerweise die Zelle induziert, zu einem nicht-neuronalen Phänotyp zu differenzieren, wobei das Mittel ein Follistatin-Protein ist und wobei das Verfahren nicht die Verwendung von humanen embryonalen Zellen beinhaltet.

11. Verfahren nach Anspruch 10, wobei die Zelle weiter mit einem zweiten Wachstumsfaktor mit neurotropher Aktivität, wie beispielsweise ein Nervenwachstumsfaktor, ziliarer neurotropher Wachstumsfaktor, Schwannomabgeleiteter Wachstumsfaktor, glialer Wachstumsfaktor, striatal abgeleiteter neuronotropher Faktor, Plättchen-abgelciteter Wachstumsfaktor, Streufaktor, ein Wirbeltier-*Hedgehog*-Protein, Noggin und ein Ligand für einen *Notch-*Rezeptor, in Kontakt gebracht wird.

12. Verfahren nach Anspruch 10, wobei die neuronale Vorläuferzelle eine humane Zelle ist.

13. Verfahren zum Induzieren einer Vorläuferzelle, entlang eines bestimmten neuronalen Weges *in vitro* zu differenzieren, umfassend Inkontaktbringen der Zelle mit einem Mittel, das einen Activin-Signalweg in der Zelle unterbricht, wobei der Signalweg normalerweise die Zelle induziert, zu einem nicht-neuronalen Zelltyp zu differenzieren, wobei das Mittel ein Follistatin-Protein ist und wobei das Verfahren nicht die Verwendung von humanen embryonalen Zellen beinhaltet.

14. Kit zum Induzieren einer normalen, nicht-neoplastischen Zelle, entlang eines bestimmten neuronalen Weges zu differenzieren, umfassend
(i) eine pharmazeutische Formulierung eines Mittels, das die Activin-Signalgebung in der Zelle antagonisiert, wobei das Mittel ein Follistatin-Protein ist, und
(ii) eine pharmazeutische Formulierung eines Wachstumsfaktors mit neurotropher oder neuraler induktiver Aktivität,
wobei das Mittel und der Wachstumsfaktor in Mengen bereitgestellt werden, die ausreichend sind, um die Differenzierung der Zelle zu einem Phänotyp einer neuronalen Zelle zu induzieren oder aufrecht zu erhalten.

15. Kit nach Anspruch 14, wobei der Wachstumsfaktor aus einem Nervenwachstumsfaktor, ziliaren neurotrophen Wachstumsfaktor, Schwannomabgeleiteten Wachstumsfaktor, glialen Wachstumsfaktor, striatal abgeleiteten neuronotrophen Faktor, Plättchen-abgeleiteten Wachstumsfaktor, Streufaktor, einem Wirbeltier-Hedgehog-Protein, Noggin und einem Liganden für einen *Notch*-Rezeptor ausgewählt ist.

16. Kit nach Anspruch 14, wobei das Mittel die Bindung von Activin mit Activin-Rezeptoren durch seine eigene Bindung an die Activin-Rezeptoren hemmt.

17. Verwendung eines Mittels, das Activin-induzierte Signalübertragung antagonisiert, bei der Herstellung eines Medikaments zum Bewirken, *in vivo,* der Erhaltung, funktionellen Leistung und Alterung von normalen neuronalen Zellen eines erwachsenen Patienten und Reparatur und Regeneration von chemisch oder mechanisch verletzten neuronalen Zellen, wobei das Mittel ein Follistatin-Protein ist.

18. Verwendung einer therapeutisch wirksamen Menge eines Mittels, das die biologische Wirkung von Activin antagonisiert, für die Herstellung eines Medikaments zum Behandeln einer degenerativen Erkrankung des Nervensystems, **gekennzeichnet durch** neuronalen Zelltod bei einem erwachsenen Patienten, wobei das Activin normalerweise Zellen in dem Patienten induziert, zu einem nicht-neuronalen Phänotyp zu differenzieren, wobei das Mittel ein Follistatin-Protein ist.

19. Verwendung nach Anspruch 18, wobei die therapeutisch wirksame Menge des Mittels die Dedifferenzierung von neuronalen Zellen des Patienten hemmt.

20. Verwendung nach Anspruch 18, wobei die therapeutisch wirksame Menge des Mittels die terminale Differenzierung von Zellen des Patienten zu einem Phänotyp einer neuralen Zelle induziert.

21. Verwendung nach Anspruch 20, wobei der Phänotyp der neuralen Zelle eine Gliazelle ist.

22. Verwendung nach Anspruch 20, wobei der Phänotyp der neuralen Zelle eine Nervenzelle ist.

23. Verwendung nach Anspruch 18, wobei die degenerative Erkrankung eine neuromuskuläre Erkrankung ist.

24. Verwendung nach Anspruch 18, wobei die degenerative Erkrankung eine autonome Erkrankung ist.

25. Verwendung nach Anspruch 18, wobei die degenerative Erkrankung eine Erkrankung des zentralen Nervensystems ist.

26. Verwendung nach Anspruch 18, wobei die degenerative Erkrankung aus Alzheimer-Krankheit, Parkinson-Krankheit, amyotropher Lateralsklerose, Pick-Krankheit, Huntington-Krankheit, multipler Sklerose, neuronaler Schädigung, resultierend aus Anoxie-Ischämie, neuronaler Schädigung, resultierend aus Trauma, und neuronaler Degeneration, verbunden mit einem natürlichen Alterungsprozeß, ausgewählt ist.

27. Verwendung nach Anspruch 18, wobei das Medikament weiter eine therapeutisch wirksame Menge eines zweiten Wachstumsfaktors mit neurotropher Aktivität einschließt.

28. Verwendung nach Anspruch 27, wobei der zweite Wachstumsfaktor aus einem Nervenwachstumsfaktor, ziliaren neurotrophen Wachstumsfaktor, Schwannomabgeleiteten Wachstumsfaktor, glialen Wachstumsfaktor, striatal abgeleiteten neuronotrophen Faktor, Plättchen-abgeleiteten Wachstumsfaktor ausgewählt ist.

29. Verwendung nach Anspruch 18, wobei das Medikament weiter eine therapeutisch wirksame Menge eines antimitotischen Mittels einschließt.

30. Verwendung nach Anspruch 29, wobei das antimitotische Mittel aus Cytosin, Arabinosid, 5-Fluoruracil, Hydroxyhamstoff und Methotrexat ausgewählt ist.

31. Verwendung nach Anspruch 18, wobei das Medikament zur Verabreichung an einen Menschen formuliert ist.

## Revendications

1. Procédé d'induction de différenciation d'une cellule souche vers un phénotype de cellule neuronale *in vitro,* comprenant la mise en contact de ladite cellule avec un agent qui agit comme antagoniste de l'action biologique de l'activine, ladite activine induisant habituellement la différenciation de ladite cellule vers un phénotype non neuronal, où ledit agent est une protéine follistatine et où ladite cellule souche est une cellule autre qu'une cellule embryonnaire humaine.

2. Procédé selon la revendication 1, dans lequel ledit agent inhibe l'activité biologique de l'activine en empêchant ladite activine de se lier aux récepteurs d'activine à la surface de ladite cellule.

3. Procédé selon la revendication 2, dans lequel ledit agent se lie à l'activine et séquestre l'activine de telle façon qu'elle ne peut pas se lier auxdits récepteurs d'activine.

4. Procédé selon la revendication 1, dans lequel ladite cellule est une cellule humaine.

5. Procédé selon la revendication 2, dans lequel ledit agent inhibe la liaison de l'activine aux récepteurs d'activine *via* sa propre liaison auxdits récepteurs d'activine.

6. Procédé selon la revendication 1, dans lequel ladite cellule est en outre mise en contact avec un second facteur de croissance ayant une activité d'induction neurale ou neurotrophique, tel qu'un facteur de croissance nerveux, un facteur de croissance neurotrophique ciliaire, un facteur de croissance dérivé de schwannome, un facteur de croissance glial, un facteur neuronotrophique dérivé de striatum, un facteur de croissance dérivé des plaquettes, un facteur de croissance des hépatocytes, une protéine *hedgehog* de vertébré, noggin, et un ligand d'un récepteur *Notch.*

7. Procédé selon la revendication 1, dans lequel ladite cellule est une cellule progénitrice neurale.

8. Procédé selon la revendication 1, dans lequel ladite cellule est sélectionnée parmi une cellule progénitrice de mélanocyte, une cellule progénitrice gliale, une cellule progénitrice de neurone sensoriel, une cellule progénitrice sympatho-surrénale, une cellule progénitrice parasympathique, et une cellule progénitrice entérique.

9. Procédé selon la revendication 1, dans lequel ladite cellule est sélectionnée parmi une cellule embryonnaire, une cellule foetale, et une cellule néonatale, et dans lequel ladite cellule embryonnaire est une cellule embryonnaire non-humaine.

10. Procédé de prévention de la mort d'une cellule progénitrice neuronale *in vitro,* comprenant la mise en contact de ladite cellule avec un agent qui agit comme antagoniste de l'action biologique de l'activine, ladite activine induisant habituellement la différenciation de ladite cellule vers un phénotype non neuronal, où ledit agent est une protéine follistatine et où ledit procédé n'implique pas l'utilisation de cellules embryonnaires humaines.

11. Procédé selon la revendication 10, dans lequel ladite cellule est en outre mise en contact avec un second facteur de croissance ayant une activité neurotrophique, tel qu'un facteur de croissance nerveux, un facteur de croissance neurotrophique ciliaire, un facteur de croissance dérivé de schwannome, un facteur de croissance glial, un facteur neuronotrophique dérivé de striatum, un facteur de croissance dérivé des plaquettes, un facteur de croissance des hépatocytes, une protéine *hedgehog* de vertébré, noggin, et un ligand d'un récepteur *Notch.*

12. Procédé selon la revendication 10, dans lequel la cellule progénitrice neuronale est une cellule humaine.

13. Procédé d'induction de la différenciation d'une cellule progénitrice le long d'une voie déterminée neuronale *in vitro,* comprenant la mise en contact de ladite cellule avec un agent qui interrompt une voie de signalisation activine dans ladite cellule, ladite voie de signalisation induisant habituellement la différenciation de ladite cellule vers un type cellulaire non neuronal, où ledit agent est une protéine follistatine et où ledit procédé n'implique pas l'utilisation de cellules embryonnaires humaines.

14. Kit d'induction d'une différenciation de cellule normale, non néoplasique le long d'une voie neuronale déterminée, comprenant:
(i) une formulation pharmaceutique d'un agent qui agit comme antagoniste de la signalisation activine dans ladite cellule, où ledit agent est une protéine follistatine, et
(ii) une formulation pharmaceutique d'un facteur de croissance ayant une activité d'induction neurale ou neurotrophique,
où lesdits agent et facteur de croissance sont fournis dans des quantités suffisantes pour induire ou maintenir une différenciation de ladite cellule en un phénotype de cellule neuronale.

15. Kit selon la revendication 14, où ledit facteur de croissance est sélectionné parmi un facteur de croissance nerveux, un facteur de croissance neurotrophique ciliaire, un facteur de croissance dérivé de schwannome, un facteur de croissance glial, un facteur neuronotrophique dérivé de striatum, un facteur de croissance dérivé des plaquettes, un facteur de croissance des hépatocytes, une protéine *hedgehog* de vertébré, noggin, et un ligand d'un récepteur *Notch.*

16. Kit selon la revendication 14, dans lequel ledit agent inhibe la liaison de l'activine aux récepteurs d'activine via sa propre liaison auxdits récepteurs d'activine.

17. Utilisation d'un agent qui agit comme antagoniste d'une transduction de signal induite par de l'activine dans la fabrication d'un médicament pour réaliser, *in vivo,* le maintien, la performance fonctionnelle, et le vieillissement de cellules neuronales normales d'un patient adulte, et la réparation et la régénération de cellules neuronales ayant subies des lésions mécaniques ou chimiques, dans laquelle ledit agent est une protéine follistatine.

18. Utilisation d'une quantité thérapeutiquement efficace d'un agent qui agit comme antagoniste de l'action biologique de l'activine pour la préparation d'un médicament pour traiter un trouble dégénératif du système nerveux **caractérisé par** la mort de cellule neuronale chez un patient adulte, ladite activine induisant habituellement la différenciation chez ledit patient de cellules vers un phénotype non neuronal, où ledit agent est une protéine follistatine.

19. Utilisation selon la revendication 18, dans laquelle ladite quantité thérapeutiquement efficace dudit agent inhibe la dé-différenciation de cellules neuronales dudit patient.

20. Utilisation selon la revendication 18, dans laquelle ladite quantité thérapeutiquement efficace dudit agent induit la différenciation terminale de cellules dudit patient vers un phénotype de cellule neurale.

21. Utilisation selon la revendication 20, dans laquelle ledit phénotype de cellule neurale est une cellule gliale.

22. Utilisation selon la revendication 20, dans laquelle ledit phénotype de cellule neurale est une cellule nerveuse.

23. Utilisation selon la revendication 18, dans laquelle ledit trouble dégénératif est un trouble neuromusculaire.

24. Utilisation selon la revendication 18, dans laquelle ledit trouble dégénératif est un trouble autonome.

25. Utilisation selon la revendication 18, dans laquelle ledit trouble dégénératif est un trouble du système nerveux central.

26. Utilisation selon la revendication 18, dans laquelle ledit trouble dégénératif est sélectionné parmi la maladie d'Alzheimer, la maladie de Parkinson, une sclérose latérale amyotrophique, la maladie de Pick, la maladie de Huntington, une sclérose multiple, un dommage neuronal résultant d'une anoxie-ischémie, un dommage neuronal résultant d'un trauma, et une dégénérescence neuronale associée à un processus de vieillissement naturel.

27. Utilisation selon la revendication 18, dans laquelle ledit médicament inclut en outre une quantité thérapeutiquement efficace d'un second facteur de croissance ayant une activité neurotrophique.

28. Utilisation selon la revendication 27, dans laquelle ledit second facteur de croissance est sélectionné parmi un facteur de croissance nerveux, un facteur de croissance neurotrophique ciliaire, un facteur de croissance dérivé de schwannome, un facteur de croissance glial, un facteur neuronotrophique dérivé de striatum, un facteur de croissance dérivé des plaquettes.

29. Utilisation selon la revendication 18, dans laquelle ledit médicament inclut en outre une quantité thérapeutiquement efficace d'un agent antimitotique.

30. Utilisation selon la revendication 29, dans laquelle ledit agent antimitotique est sélectionné parmi de la cytosine, de l'arabinoside, du 5-fluorouracile, de l'hydroxyurée, et du méthotrexate.

31. Utilisation selon la revendication 18, dans laquelle le médicament est formulé pour être administré à un humain.
